# EUROPÄISCHE PATENTSCHRIFT

(11) **EP 2 304 018 B1**
(45) Veröffentlichungstag und Bekanntmachung des Hinweises auf die Patenterteilung: **30.11.2011**
(21) Anmeldenummer: 09768604.2
(22) Anmeldetag: 24.06.2009
(51) Int. Cl.: C12M 1/107

(54) **Erdbeckenfermenter**
Ground basin fermenter
Fermenteur du type bassin enterré

(30) Priorität: 25.06.2008 AT 10142008
(43) Veröffentlichungstag der Anmeldung: 06.04.2011
(60) Teilanmeldung: 11001063.4
(73) Patentinhaber: Sattler AG, 8041 Graz (AT)
(72) Erfinder: WIEDAU, Helmut, 48161 Münster (DE); VERWANGER, Peter, A-8041 Graz (AT)
(74) Vertreter: Ellmeyer, Wolfgang
(86) Internationale Anmeldenummer: PCT/AT2009/000254
(87) Internationale Veröffentlichungsnummer: WO 2009/155629

(56) Entgegenhaltungen:
- EP-A- 0 014 136
- WO-A-98/28402
- DE-U1-202005 011 689
- FR-A- 2 519 965

## Beschreibung

Die Erfindung betrifft einen Erdbeckenfermenter mit einem abgesenkten Beckenboden, der mit einer Dichtungsschicht abgedeckt ist und ein Erdbecken ausbildet, und an dessen Beckenkrone zumindest eine gasdichte Abdeckung festgelegt ist, wobei Zu- und Ableitungen und zumindest ein Rührwerk vorgesehen sind, wobei zumindest eine Auflagerstelle für das zumindest eine Rührwerk oder für zumindest ein Führungs- oder Abstützelement des zumindest einen Rührwerks im Wesentlichen auf der Höhe der Beckenkrone oder darüber und innerhalb des von der Beckenkrone umschlossenen Bereichs vorgesehen ist.

Erdbeckenfermenter zur Biogasproduktion dienen zumeist dem Vergären von Gülle oder hochbelasteten Abwässern und erlauben aufgrund ihrer Bauweise eine sehr kostengünstige Errichtung und ein einfaches Betreiben.

Die meisten Erdbeckenfermenter sind im Wesentlichen aus Erdbecken gebildet, die ein- oder zweischalig mit Folien ausgekleidet und mit einer gasdichten, durch den Gasdruck gestützten Abdeckung oben abgeschlossen sind, wobei die Stabilität der Abdeckung durch Vorsehen eines Ballasts gewährleistet ist Das Rühren erfolgt, wenn überhaupt, hydraulisch durch Umwälzen des Substrates mit externen Pumpen oder durch das Einblasen von Biogas am Fermenterboden.

Bei Einsatz von mechanischen Rührwerken ergeben sich besondere Anforderungen hinsichtlich der Lagerung. Mechanische Rührwerke erzeugen erhebliche Auflagerkräfte, die bei konventionellen Fermenterbehältern über den Behälterboden, die starre Behälterwand oder die starre Behälterdecke abgetragen werden. Da der dahinterliegende Erdwall zwar die Belastung des Fermenterinhaltes nicht jedoch die punktuellen Lasten eines Rührwerkes aufnehmen kann, sind Durchdringungen für den Rührwerksantrieb und Auflagerungen am Erdwall eines Erdbeckens nur mit aufwendigen zusätzlichen Stützkonstruktionen, z.B. aus Stahl oder Beton zu bewerkstelligen, die z.B. zu Setzungen und damit zu einer Strapazierung der Erdbeckenabdichtung führen können.

Die Durchdringungen stellen darüber hinaus Unterbrechungen der Abdichtungsfolien dar und müssen entsprechend dicht, statisch aufnahmefähig und für Wartungsarbeiten zugänglich sein, da die Beständigkeit der auf dem Beckenboden aufgebrachten Dichtungsschicht von besonderer Wichtigkeit ist. Leckstellen können zu einem unbemerkten Eindringen des zu verarbeitenden Substrats in das darunter liegende Erdreich führen und dies soll schon allein aus Gründen des Umweltschutzes, insbesondere wegen der drohenden Grundwasserverseuchung unterbunden werden. Selbst wenn die Leckstellen rasch gefunden bzw. detektiert werden können, bedeutet das Erfordernis der Sanierung des Beckenbodens einen übermäßigen Aufwand, der relativ lange Standzeiten mit sich bringt. Die dauerhafte Abdichtung des Beckenbodens ist somit eine weitere Schwachstelle der bisher bekannten Erdbeckenfermenter.

Damit gehen aber die wesentlichen Vorteile der Erdbeckenfermenter wie z.B. die niedrigen Kosten, die einfache Bauart und die hohe Betriebssicherheit verloren.

In der FR 2 519 965 A ist ein Erdbecken mit einer gasdichten Abdeckung und einer im Inneren desselben angeordneten Brücke gezeigt, auf der ein nach unten ragendes Rührwerk gelagert ist. Sollen Arbeiten am Rührwerk oder an dessen Lagerung durchgeführt werden, so muss zu diesem Zweck ein Zutritt in den Gasraum unterhalb der gasdichten Abdeckung erfolgen, was wegen der Explosionsgefahr nur bei einer Betriebsunterbrechung geschehen kann.

Aufgabe der Erfindung ist es daher, einen Erdbeckenfermenter der eingangs genannten Art anzugeben, der bei einfacher Bauweise einen sicheren und dichten Betrieb ermöglicht.

Weiteres Ziel der Erfindung ist es, einen Erdbeckenfermenter zu schaffen, der die Auflagerkräfte für Rührwerke unterschiedlichster Bauart aufnehmen kann, ohne dabei die Dichtheit und Betriebssicherheit zu beeinträchtigen.

Erfindungsgemäß wird dies dadurch erreicht, dass die zumindest eine Auflagerstelle außerhalb der zumindest einen gasdichten Abdeckung vorgesehen ist, wobei ein Antriebselement des zumindest einen Rührwerks oder das zumindest eine Führungsoder Abstützelement des zumindest einen Rührwerks gasdicht durch eine Öffnung in der zumindest einen gasdichten Abdeckung hindurchgeführt ist.

Im Unterschied zu den bisher bekannten Lösungen wird somit zumindest eine Auflagerstelle für das Rührwerk geschaffen, die sich oberhalb des Substratfüllstandes befindet, wodurch Probleme mit der Tragfähigkeit oder Belastbarkeit des Erdbeckens vermieden werden können. Des weiteren ermöglicht die erfindungsgemäße Anordnung von Auflagerstellen eine dauerhafte und stabile Lagerung des Rührwerks und zugleich die Möglichkeit eines einfachen Zugriffes auf zu wartende Komponenten. Die konstruktive Realisierung eines Aufbaus, der die erfindungsgemäße Anordnung der zumindest einen Auflagerstelle ermöglicht, kann unterschiedlich geschehen und wird nachstehend in weiteren Ausführungsvarianten der Erfindung beispielhaft gezeigt.

Weiters ist erfindungsgemäß zum Zwecke der besseren Zugänglichkeit zum Rührwerk oder Teilen desselben zumindest eine Auflagerstelle außerhalb der zumindest einen gasdichten Abdeckung vorgesehen, wobei ein Antriebselement des zumindest einen Rührwerks oder das zumindest eine Führungs- oder Abstützelement des zumindest einen Rührwerks gasdicht durch eine Öffnung in der zumindest einen gasdichten Abdeckung hindurchgeführt ist.

Als Führungselement kann eine Führungsschiene ausgebildet sein, die mit ihrem unteren Ende in einem Fundament am Beckenboden verankert ist und mit ihrem oberen Ende, das durch die gasdichte Abdeckung hindurchgeführt ist, auf der zumindest einen Auflagerstelle abgestützt ist. Auf diese Weise ist das Führungselement an beiden Enden ausreichend stabil fixiert, um die Auflagerkräfte des Rührwerkes aufnehmen zu können.

Eine weitere Variante der Erfindung kann darin bestehen, dass die Dichtungsschicht des Beckenbodens durchdringungsfrei ausgeführt ist, und dass die Zu- und Ableitungen oberhalb des Beckenbodens und innerhalb der Beckenkrone in das Innere des Erdbeckens geführt sind. Die auf diese Weise vermiedenen Unterbrechungen der Dichtungsschicht ermöglichen eine deutliche Reduktion von fehlerhaften Stellen in der Abdichtung gegenüber dem Untergrund. Setzungen im Erdreich haben dadurch keine negativen Auswirkungen auf die Dichthaltigkeit.

Da sämtliche Ab- und Zuleitungen sowie mechanische Kopplungen und Führungen von oben durch die Gasmembran geführt sind, ist kein Durchstoßen des Beckenbodens erforderlich. Die Dichtungsschicht kann daher ohne die Gefahr einer Riss- oder Leckbildung im Bereich von Durchdringungen für Zu- und Ableitungen ausgeführt werden. Die komplette Bedienung sowie die Gasableitung, Substratzu- und -abführung erfolgen somit ausschließlich vom Niveau der Beckenkrone aus oder oberhalb desselben, also oberhalb des höchsten Substrat-Füllstandes. Aufgrund der durchgängigen, also ohne Durchdringung versehenen Dichtungsschicht bis zur Oberkante des Erdbeckens ist die Gefahr einer Undichtheit bzw. der Gefährdung praktisch vollkommen beseitigt. Weiters besteht auch keine Verstopfungsgefahr der Gas-Ableitung durch Substraterhöhung oder Schaumbildung, weil die Gas-Ableitung weit oberhalb der Substratoberfläche angeordnet ist.

Weiters ist die Gefahr einer Rissbildung durch Bodensetzungen eliminiert, weil z.B. eine hochelastische, zweischalige Dichtungsschicht durch Aufbringen von PE-Dichtungsbahnen in Deponiequalität vorgesehen sein kann.

Eine weitere Ausführungsform der Erfindung kann darin bestehen, dass eine Bedienbrücke vorgesehen ist, die das Erdbecken überspannt, und dass die zumindest eine Auflagerstelle auf der Bedienbrücke ausgebildet ist, welche selbst hohe Auflagerkräfte aufnehmen kann. Der Betrieb und die Wartung von Rührwerken sind auf diese Weise einfach und sicher möglich.

Weiters können in der Bedienbrücke ein oder mehrere Durchlässe vorgesehen sein, durch welche die Zu- und Ableitungen und/oder das Führungs- oder Abstützelement und/oder das Antriebselement des Rührwerks gasdicht in das darunter liegende Erdbeckeh hindurchgeführt sind.

Sämtliche Wartungs- und Bedienfunktionen lassen sich über diese Bedienbrücke oberhalb des höchsten Substrat-Füllstandes ausführen und erlauben damit nicht nur eine einfache Einflussnahme auf das darunter liegende Erdbecken sondern auch eine gute Anpassbarkeit an die gewünschten Konstruktionsmerkmale.

Eine weitere Verbesserung des Zugriffes auf das Innere des Erdbeckens lässt sich erzielen, indem auf der Bedienbrücke zumindest ein dichtend verschließbarer Service-Schacht mit einer Schachtöffnung angeordnet ist, in dem die Auflagerstelle für das zumindest eine Führungs- oder Abstützelement des Rührwerks vorgesehen ist, welches entlang des Führungs- oder Abstützelements höhenverstellbar ist. Damit können nicht nur die Möglichkeiten der Steuerbarkeit des Rührwerkes erhöht werden, sondern es kann auch der Austausch oder die Wartung des Rührwerkes von oben aus über die Bedienbrücke erfolgen.

Gemäß einer weiteren Ausbildung der Erfindung kann der Service-Schacht mit einer Zu- oder Ableitung verbunden sein, die mit dem Innenraum des Erdbeckenfermenters kommuniziert. Die Gas-Ableitung erfolgt damit auf dem höchsten Punkt der gesamten Anordnung, wodurch Probleme mit dem Überschäumen oder Überquellen des Substrates vermieden werden.

Wenn die Bedienbrücke gemäß einer weiteren Ausführungsform der Erfindung begehbar ist, können Wartungs- und Bedienarbeiten direkt von der Bedienbrücke aus - ohne Notwendigkeit eines Gerüsts oder Krans und somit ohne Sicherheitsgefährdung des Personals - ausgeführt werden.

Um ausreichende statische Sicherheit zu gewährleisten kann die Bedienbrücke über zumindest eine Stütze gegenüber dem Beckenboden abgestützt sein.

Eine weitere Möglichkeit des direkten Zutritts in das Innere des Erdbeckens oder dessen Sichtung kann dadurch geschaffen werden, dass in der Bedienbrücke ein Sichtfenster ausgenommen ist, das gasdicht verschließbar ist. Über dieses Sichtfenster können Beobachtungen der Vorgänge im Inneren sowie Servicearbeiten ausgeführt werden.

Die Bedienbrücke kann zumindest einen Träger und eine Bodenabdeckplatte umfassen, wodurch ein konstruktiv einfacher Aufbau gewährleistet ist.

Von Vorteil ist es weiters, wenn die Bedienbrücke selbst als Teil der gasdichten

Abdeckung des erfindungsgemäßen Erdbeckenfermenters fungiert. Dies kann gemäß einer weiteren Ausführungsform der Erfindung dadurch realisiert sein, dass die Bedienbrücke mit ihrer Bodenabdeckplatte einen Abschnitt der gasdichten Abdeckung ausbildet, und dass die zumindest eine Gasmembran oder Gasmembranteile mit der Bedienbrücke gasdicht verbunden sind, sodass die Bedienbrücke zusammen mit der Gasmembran oder den Gasmembranteilen die gasdichte Abdeckung des Erdbeckens ausbilden. Auf diese Weise können die zur Unsetzung der Erfindung erforderlichen Durchlässe für die Durchführung der Zu- und Ableitungen bzw. der mechanischen Kopplung und/oder Führung auf einfache Weise in der Bodenabdeckplatte ausgeführt sein. Dies ermöglicht eine sehr einfache konstruktive Variierungsmöglichkeit beim Aufbau des erfindungsgemäßen Erdbeckenfermenters.

Eine dauerhafte Abdichtung zwischen jener Berandung der Gasmembran, die an die Bedienbrücke angrenzt, lässt sich gemäß einer Weiterbildung der Erfindung dadurch erzielen, dass die gasdichte Verbindung zwischen der Bedienbrücke und der zumindest einen Gasmembran oder den Gäsmembranteilen durch eine gasdichte Klemmung im Randbereich der Bedienbrücke gebildet ist.

Eine alternative Ausführungsform zur Bedienbrücke kann dadurch gegeben sein, dass ein Bediensteg vorgesehen ist, der an einem Ende im Bereich der Beckenkrone befestigt ist und mit seinem freien Ende in das Erdbecken ragt, wobei die zumindest eine Auflagerstelle auf dem Bediensteg ausgebildet ist, der für eine ausreichende Abstützung der Auflagerstelle sorgt.

Weiters können auf dem Bediensteg ein oder mehrere Durchlässe vorgesehen sein, durch welche die Zu- und Ableitungen gasdicht in das darunter liegende Erdbecken hindurchgeführt sind. Im Gegensatz zur Bedienbrücke benötigt der Bediensteg nur eine Festlegung auf einer Seite des erfindungsgemäßen Erdbeckenfermenters, weil er mit seinem freien Ende in das Erdbecken ragt ohne auf der gegenüberliegende Seite auf der Beckenkrone aufzuliegen.

Unter Ausnutzung der gleichen, bereits im Zusammenhang mit der Bedienbrücke genannten Vorteile kann auf dem Bediensteg zumindest ein dichtend verschließbarer Service-Schacht mit einer Schachtöffnung angeordnet sein, in dem die Auflagerstelle für das zumindest eine Führungs- oder Abstützelement des Rührwerks vorgesehen ist, welches entlang des Führungs- oder Abstützelements höhenverstellbar ist.

Weiters kann der Service-Schacht in gleicher Weise wie bei der Bedienbrücke mit einer Zu- oder Ableitung verbunden sein, die mit dem Innenraum des Erdbeckenfermenters kommuniziert.

Auch der Bediensteg kann für Bedien- und Wartungsarbeiten begehbar sein. Für eine sichere Abstützung des freien Endes des Bediensteges kann dieser über zumindest eine Stütze gegenüber dem Beckenboden abgestützt sein. Für eine Verbesserung des Zugriffes auf das Innere des Erdbeckens bzw. zum Zwecke der Beobachtung desselben kann im Bediensteg ein Sichtfenster ausgenommen sein, das gasdicht verschließbar ist. Für Wartungs- und Reparaturarbeiten können Bedienpersonen durch das geöffnete Sichtfenster in das Innere des Erdbeckens gelangen.

Ein einfacher konstruktiver Aufbau des Bediensteges und eine einfache Montage desselben lässt sich erreichen, wenn dieser zumindest einen Träger und eine Bodenabdeckplatte umfasst.

Eine weitere Ausführungsform der Erfindung kann darin bestehen, dass der Bediensteg mit seiner Bodenabdeckplatte einen Abschnitt der gasdichten Abdeckung ausbildet, und dass die zumindest eine Gasmembran oder Gasmembranteile mit dem Bediensteg gasdicht verbunden sind, sodass der Bediensteg zusammen mit der Gasmembran oder den Gasmembranteilen die gasdichte Abdeckung des Erdbeckens ausbilden. Auf diese Weise lassen sich sämtliche Zu- und Ableitungen und alte erforderlichen mechanischen Kopplungen über die Bodenabdeckplatte des Bediensteges in das Innere des Erdbeckens führen, sodass die Bodenabdeckplatte bei unveränderter Gasmembran jederzeit ausgetauscht oder konstruktiv anders ausgeführt werden kann.

Eine zuverlässige und dauerhafte Einbindung des Bediensteges in die gasdichte Abdeckung des erfindungsgemäßen Erdbeckenfermenters lässt sich erreichen, wenn in weiterer Ausbildung der Erfindung die gasdichte Verbindung zwischen dem Bediensteg und der zumindest einen Gasmembran oder den Gasmembranteilen durch eine gasdichte Klemmung im Randbereich der Bodenabdeckplatte gebildet ist.

Nachfolgend wird die Erfindung anhand der in den Zeichnungen dargestellten Ausführungsbeispiele eingehend erläutert. Es zeigt dabei
Fig.1 einen Schnitt durch eine Ausführungsform des erfindungsgemäßen Erdbeckenfermenters;
Fig.2 einen Schnitt durch eine weitere Ausführungsform des erfindungsgemäßen Erdbeckenfermenters;
Fig.3 einen Schnitt durch eine weitere Ausführungsform des erfindungsgemäßen Erdbeckenfermenters;
Fig.4 einen Schnitt durch ein Detail einer weiteren Ausführungsform des erfindungsgemäßen Erdbeckenfermenters;
Fig.5 einen Schnitt durch ein Detail einer weiteren Ausführungsform des erfindungsgemäßen Erdbeckenfermenters;
Fig.6 eine Draufsicht auf eine weitere Ausführungsform des erfindungsgemäßen Erdbeckenfermenters;
Fig.7 einen Querschnitt quer zur Längsachse des Erdbeckenfermenters gemäß Fig.6;
Fig.8 einen Längsquerschnitt des Erdbeckenfermenters gemäß Fig. 6;
Fig.9 zeigt ein Detail einer Ausführungsvariante zu Fig.6 im Schnitt;
Fig.10 zeigt ein Detail einer weiteren Ausführungsvariante zu Fig.6 im Schnitt;
Fig.11 einen Querschnitt quer zur Längsachse eines weiteren Ausführungsbeispiels des erfindungsgemäßen Erdbeckenfermenters;
Fig.12 einen Schnitt durch ein Detail des Erdbeckenfermenters gemäß Fig.11;
Fig.13 einen teilweisen Schnitt durch eine weitere Ausführungsform des erfindungsgemäßen Erdbeckenfermenters;
Fig.14 eine Draufsicht auf das Detail gemäß Fig.13;
Fig.15 einen teilweisen Schnitt durch eine weitere Ausführungsform des erfindungsgemäßen Erdbeckenfermenters;
Fig.16 eine Draufsicht auf das Detail gemäß Fig.15;
Fig.17 einen Längsschnitt durch eine weitere Ausführungsform des erfindungsgemäßen Erdbeckenfermenters;
Fig.18 einen Schnitt durch eine Variante eines Details des Erdbeckenfermenters gemäß Fig.17 und
Fig.19 einen Schnitt durch eine weitere Variante eines Details des Erdbeckenfermenters gemäß Fig.17.

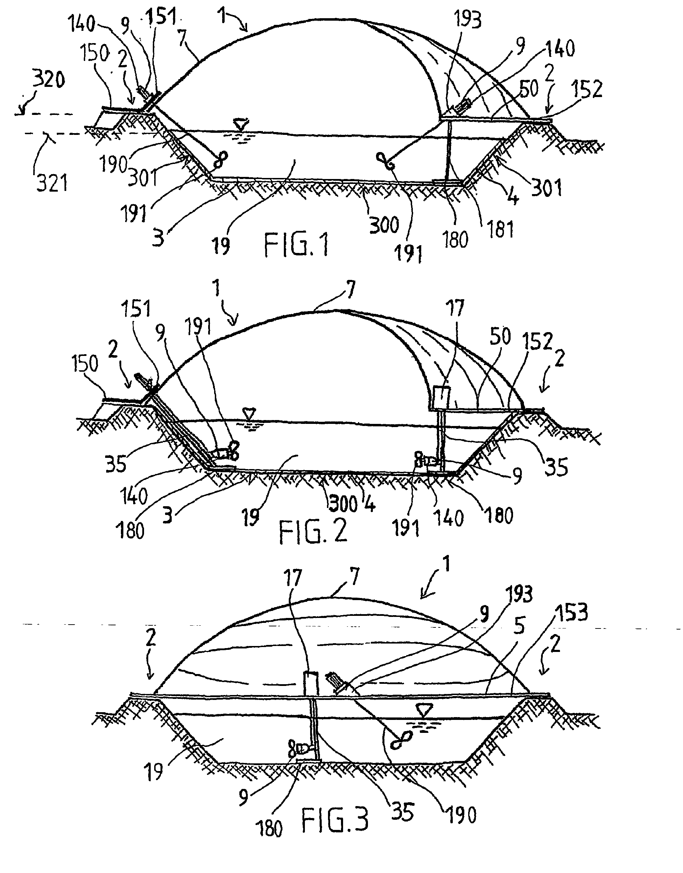

Fig.1 zeigt einen Erdbeckenfermenter 1 mit einem abgesenkten Beckenboden 3, der seitliche Wandschrägen 301 sowie eine tiefliegende Beckensohle 300 aufweist und der mit einer Dichtungsschicht 4 abgedeckt ist und ein Erdbecken 19 ausbildet, an dessen Beckenkrone 2 eine gasdichte Abdeckung, zum größten Teil in Form einer Gasmembran 7, festgelegt ist. Die Gasmembran 7 wird im Betrieb durch den aufgrund der Fermentation sich ausbildenden Innen-Gasdruck in einem mehr oder weniger stark aufgeblähten Zustand vorliegen. Es ist somit in diesem Ausführungsbeispiel die gasdichte Abdeckung ohne zusätzliche Tragekonstruktion sondern nur durch die Gasmembran 7 verwirklicht, die bereichsweise mit ihrem Rand an der Beckenkrone 2 eingespannt ist.

Da das Erdbecken 19 in bestimmten Zeitabständen von außen befüllt und wieder entleert wird, sind Zu- und Ableitungen (nicht dargestellt) vorgesehen, über die Substrat, z.B. Gülle zugeführt bzw. entnommen werden kann. Der Füllstand des Substrats ist in Fig.1 auf dem mit dem Bezugszeichen 321 gezeigten Niveau. Darüber hinaus können weitere Zu- und Ableitungen in das bzw. aus dem Erdbecken 19 führen. Das bei der Fermentation entstehende Gas wird z.B. über eine Gas-Ableitung (nicht dargestellt) abgeleitet, die mit dem Innenraum des Erdbeckenfermenters 1 kommuniziert. Die Dichtungsschicht 4 kann aus einer ein- oder mehrlagigen Kunststofffolie gebildet sein.

Für ausreichende Bewegung des Erdbeckeninhalts wird durch Rührwerke 9 gesorgt, die in allen geeigneten, dem Fachmann bekannten Ausführungsformen vorliegen können.

Erfingdungsgemäß ist jeweils eine Auflagerstelle 151, 152 für die Rührwerke 9 im Wesentlichen auf der - in Fig.1 mit dem Bezugszeichen 320 versehenen - Höhe der Beckenkrone 2 und innerhalb des von der Beckenkrone 2 umschlossenen Bereichs vorgesehen. Die Anzahl der Rührwerke 9 und die Art ihrer Ausführung kann im Rahmen der Erfindung frei gewählt werden. Die Anordnung der Auflagerstellen 151, 152 kann auch oberhalb des Niveaus der Beckenkrone 2 erfolgen.

An der Auflagerstelle 151, die durch einen in Richtung zur Beckenmitte wegstehenden Schenkel eines auf der Beckenkrone 2 fixierten Lagerwinkels 150 gebildet ist, ist ein Antriebsmotor 140 des Rührwerks 9 vorgesehen, welcher über ein Antriebselement, hier eine Antriebswelle 190 eine in das Substrat eintauchende Rührschraube 191 antreibt. Die Abtriebswelle 190 ist dabei, z.B. durch eine Hohlwelle, gasdicht durch eine Öffnung der Gasmembran 7 hindurchgeführt. Durch die erfindungsgemäße Form der Anbringung der Auflagerstelle 151 können die Auflagerkräfte des Rührwerks 9 in einem ausreichenden Maß aufgenommen werden und zugleich ist die Wartung des Rührwerkes 9 auf einfache Weise möglich.

Auf der gegenüberliegenden Seite des in Fig.1 dargestellten Erdbeckenfermenters 1 ist ein weiteres Rührwerk 9 vorgesehen, dessen Antriebsmotor 140 über einen Lagerblock 193 auf einem auf der Beckenkrone 2 fixierten und von dieser in Richtung zur Mitte des Erdbeckens 19 ragenden Bediensteg 50 angeordnet ist, der eine weitere Auflagerstelle 152 ausbildet, die sich so wie die Auflagerstelle 151 außerhalb der gasdichten Abdeckung befindet. Der Bediensteg 50 ist seinerseits über eine Stütze 181 auf einem Beckenfundamentbereich 180, der auf der Beckensohle ausgebildet ist, abgestützt. Der Bediensteg 50, der einen Teil der gasdichten Abdeckung bildet, ist in dem in das Erdbecken 19 ragenden Teil randseitig mit der Gasmembran 7 gasdicht verbunden.

In dem in Fig.2 gezeigten Ausführungsbeispiel sind an gegenüberliegenden Seiten Führungselemente 35 in Form von Führungsschienen vorgesehen, entlang denen die Rührwerke 9 mit ihren Motoren 140 verschiebbar angeordnet sind, wodurch eine Höhenverstellbarkeit gegenüber der Beckensohle 300 gewährleistet ist. Die Führungselemente 35 sind an ihren unteren Enden auf jeweils einem Beckenfundamentbereich 180 aufgestützt und dort verankert, während sie mit ihren oberen Enden durch die gasdichte Abdeckung 7 hindurchgeführt und erfindungsgemäß im Wesentlichen auf der Höhe der Beckenkrone 2, und zwar auf der einen Seite an der Auflagerstelle 151 und auf der anderen Seite an dem Bediensteg 50, aufgelagert bzw. abgestützt sind. Auf dem Bediensteg 50 ist ein Schacht 17 ausgebildet, der einen Zutritt oder Zugriff durch eine auf dem Steg 50 ausgebildete Durchgangsöffnung (nicht dargestellt) in das Innere des Erdbeckenfermenters 1 ermöglicht, um eine Wartung des entlang des senkrecht verlaufenden Führungselements 35 geführten Rührwerks 9 zuzulassen.

In Fig.3 ist eine weitere Ausführungsform des erfindungsgemäßen Erdbeckenfermenters 1 gezeigt, bei der eine Bedienbrücke 5 das Erdbecken 19 zwischen gegenüberliegenden Beckenkronenbereichen überspannt und dabei eine Auflagerstelle 153 auf der Höhe der Beckenkrone 2 ausbildet, welche das Auflager für die beiden Rührwerke 9 bildet. Die Bedienbrücke 5 ist dabei so angeordnet, dass sie einen Teil der gasdichten Abdeckung des Erdbeckenfermenters 1 bildet und an ihren Rändern teilweise mit der Gasmembran 7 verbunden ist. Durch das Vorsehen der Bedienbrücke 5, die an der Beckenkrone 2 verankert ist, können die beim Betrieb auftretenden Auflagerkräfte der Rührwerke 9 so aufgenommen werden, dass ein zuverlässiger Betrieb auch bei sehr dickflüssigen Substraten durchführbar ist. Das Antriebselement 190 und das Führungselement 35 der Rührwerke 9 sind gasdicht durch eine Öffnung in der Bedienbrücke 5 hindurchgeführt.

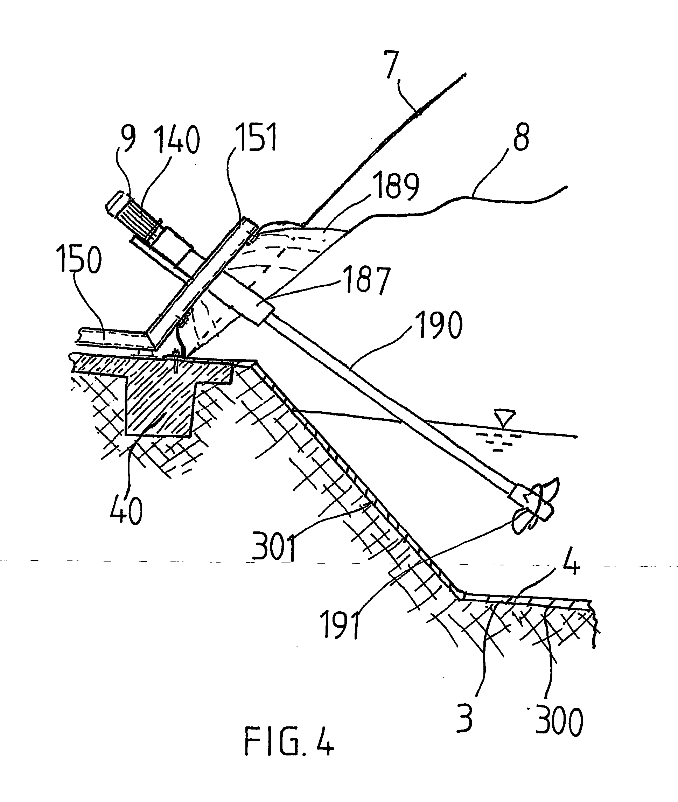

Fig. 4 zeigt die Anwendbarkeit der Erfindung für einen Erdbeckenfermenter mit der einer gasdichten Abdeckung, die durch eine Innen-Gasmembran 8 und eine Außen-Gasmembran 7 zumindest teilweise gebildet ist. Zwischen der Innen-Gasmembran 8 und der Außen-Gasmembran 7 wird über ein Stützgas ein Stützdruck aufrechterhalten, der bei sich verändernder Gasfüllung der Innen-Gasmembran 8 für eine unveränderte Gestalt der Außen-Gasmembran 7 sorgt.

Ein Hohlwellenstück 187 ist gasdicht durch sowohl die Außen-Gasmembran 7 als auch die Innen-Gasmembran 8 hindurchgeführt und ermöglicht in seinem Inneren die Rotation der über den außerhalb der gasdichten Abdeckung angebrachten Antriebsmotor 140 angetriebenen Antriebswelle 190, an deren Ende ein Rührpropeller 191 befestigt ist, der in das Substrat eintaucht. Die Auflagerstelle 151, an der das Hohlwellenstück 187 mit dem Antriebsmotor 140 festgelegt ist und welche über ein Anschlussstück 189 gasdicht mit der Innen- und Außen-Gasmembran 8, 7 verbunden ist, nimmt sämtliche im Betrieb auftretenden Auflagerkräfte auf und leitet sie über den Lagerwinkel 150 an ein Fundament 40, das in der Beckenkrone 2 eingebettet ist, ab.

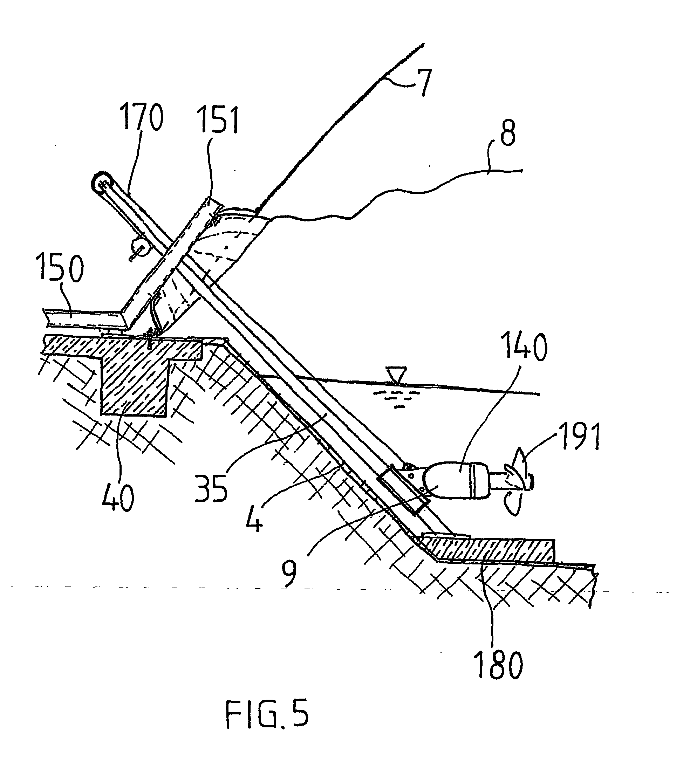

Fig.5 zeigt jene Ausführungsvariante der Erfindung, bei der die auf der Höhe der Beckenkrone oder darüber angeordnete Auflagerstelle 151 das schräg in das Becken verlaufende Führungselement 35 an seinem oberen Ende abstützt und dadurch für eine zuverlässige Höhenverstellbarkeit des Rührwerkes 9 sorgt, wobei das Führungselement 35 durch die Innen- und Außen-Gasmembran 8, 7 gasdicht hindurchgeführt ist. Über ein Seil 170 kann das Rührwerk 9 in verschiedenen Höhen im Becken angeordnet werden und bei Bedarf auch über das Niveau des Substrats gehievt werden.

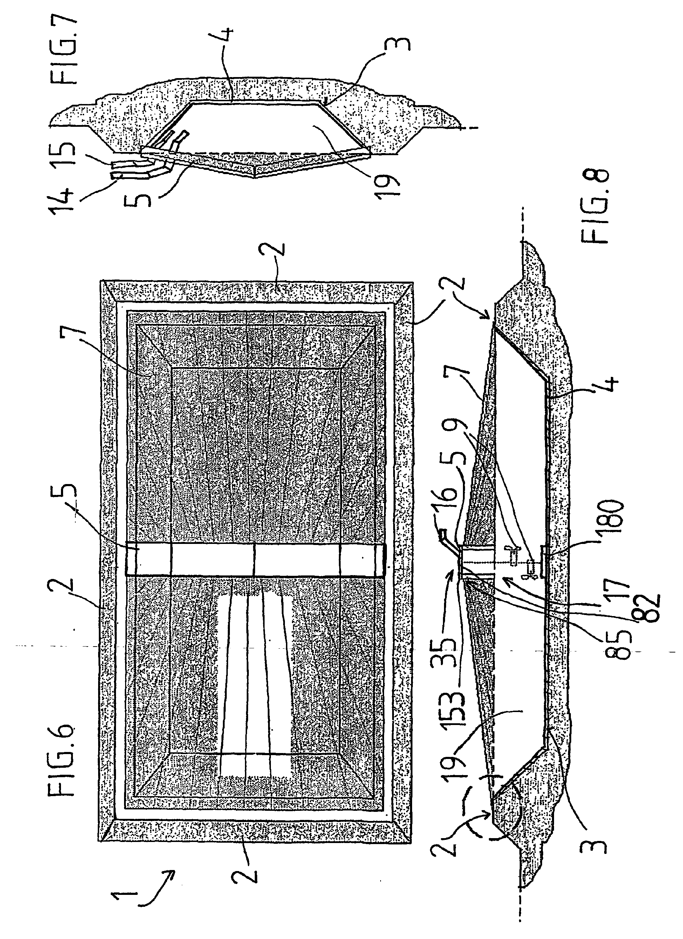

Fig.6, 7, 8 zeigen eine Aüsführüngsform des erfindungsgemäßen Erdbeckenfermenters 1, bei der die Zu- und Ableitungen 14, 15, 16 oberhalb des Beckenbodens 3 geführt sind und die obere Auflagerstelle 153 für das Führungs- und Abstützelement 35 der Rührwerke 9 auf der Höhe der Beckenkrone 2 und innerhalb des von dieser umschlossenen Bereiches vorgesehen ist. Dadurch müssen weder die schrägen Seitenwände noch die Beckensohle des Beckenbodens 3 durchstoßen werden. Somit kann die Dichtungsschicht 4 des Beckenbodens 3 durchdringungsfrei ausgeführt sein.

Um ein geeignetes Betreiben und Warten des erfindungsgemäßen Erdbeckenfermenters 1 zu ermöglichen, ist die Bedienbrücke 5 vorgesehen, die das Erdbecken 19 überspannt, wobei im gezeigten Beispiel die Bedienbrücke 5 sich von einer Längsseite des Erdbeckens 19 zur gegenüberliegenden, anderen Längsseite erstreckt und mit ihren Enden auf gegenüberliegenden Bereichen der Beckenkrone 2 aufliegt. Aus statischen Gründen ist die Bedienbrücke 5 dachartig ausgeführt, wie aus Fig.7 ersichtlich ist, sie kann aber in jeder beliebigen anderen Form gestaltet sein. Auf der Bedienbrücke 5 sind mehrere Durchlässe, durch welche die Zu- und Ableitungen 14, 15 und die Gas-Ableitung 16 gasdicht in das darunter liegende Erdbecken 19 hindurchgeführt sind, ausgebildet.

Weiters ist auf der Bedienbrücke 5 zumindest ein dichtend verschließbarer Service-Schacht 17 vorgesehen, der sich von einer Schachtöffnung in der Bedienbrücke 5 nach unten erstreckt und in dem der obere Teil des Führungs- und Abstützelements 35 für die Rührwerke 9 verläuft, welche z.B. als Stab-, Paddel- oder Tauchmotorrührwerk ausgeführt sein könnten. Die Schachtöffnung kommuniziert mit dem Inneren des Erdbeckens 19. In ihrem Bereich ist die Auflagerstelle 153 für das Führungs- und Abstützelement 35 vorgesehen, welche für die Fixierung desselben auf der Bedienbrücke vorgesehen ist.

Die Bedienbrücke 5 setzt sich im Wesentlichen aus nicht dargestellten Trägem und einer Bodenabdeckplatte 82 zusammen, wobei die Bedienbrücke 5 zusammen mit ihrer Bodenabdeckplatte 82 einen Abschnitt der gasdichten Abdeckung ausbildet. Die Gasmembran 7 besteht im Ausführüngsbeisβiel gemäß Fig.6, 7 und 8 aus zwei Gasmembranteilen, die an den an die Bodenabdeckplatte 82 angrenzenden Bereichen mit der Bedienbrücke 5 gasdicht verbunden sind, sodass die Bedienbrücke 5 zusammen mit den Gasmembranteilen die gasdichte Abdeckung des Erdbeckens 19 ausbilden. Die gasdichte Verbindung zwischen der Bedienbrücke 5 und den Gasmembranteilen ist durch eine gasdichte Klemmung im Randbereich der Bedienbrücke 5 gebildet.

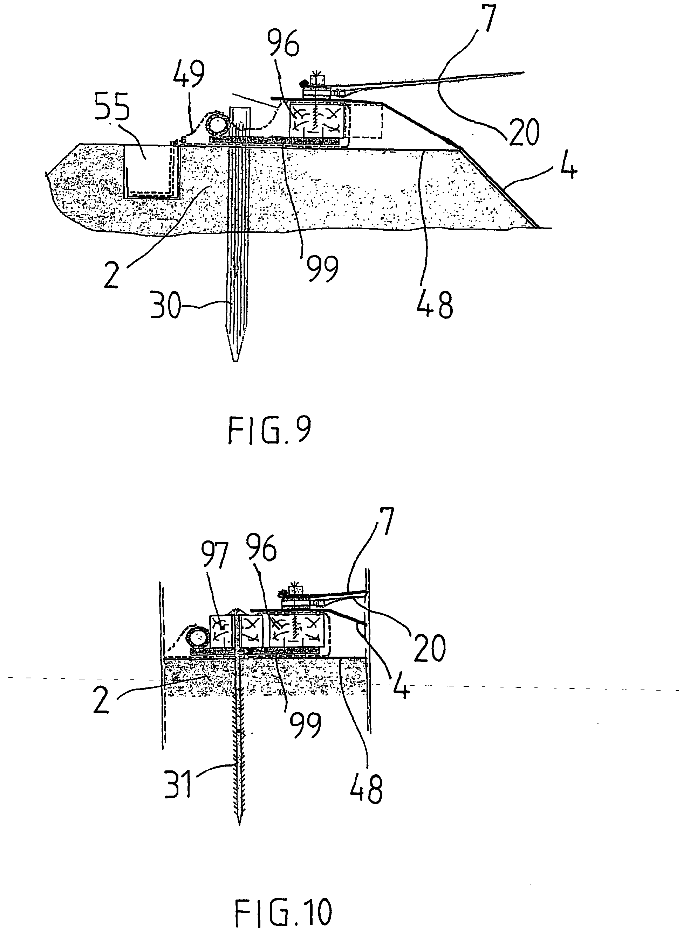

Fig.9 zeigt eine Form der Randbefestigung der Gasmembran 7, die auf einer auf der Beckenkrone 2 festgelegten Holzlatte 96 gasdicht geklemmt ist, wobei auf dieser Tragegurte 20, welche die Gasmembran 7 stützen, an ihren Enden eingehängt sind. Die Holzlatte 96 ist ihrerseits auf einer Leiste 99 befestigt, die über eine Holzpfahl-Verankerung 30 gegenüber der Beckenkrone 2 festgelegt ist. Weiters ist ein Einbindegraben 55 vorgesehen, um eine feuchtigkeits-isolierende Folie 48 und eine Nagetierschutzabdeckung 49 zu verankern.

Fig.10 zeigt eine Variante dieser Befestigungsvorrichtung, bei der durch eine weitere Latte 97 hindurchgeführte Schrauben 31 für eine sichere Verbindung mit der Beckenkrone 2 sorgen.

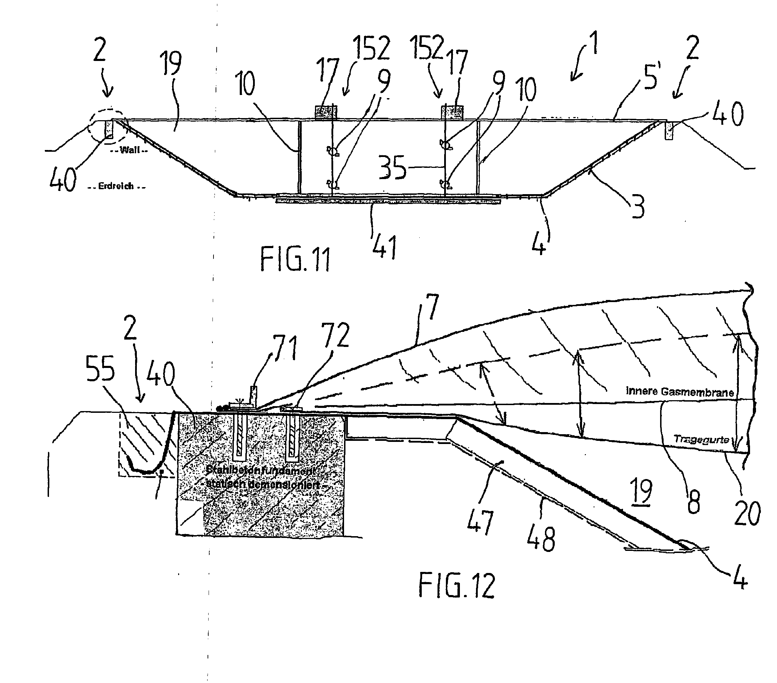

In Fig.11 und 12 ist ein weiteres Ausführungsbeispiel des erfindungsgemäßen Erdbeckenfermenters 1 gezeigt, dessen umlaufende Beckenkrone 2 durch ein Stahlbetonfundament 40 gebildet ist, welches einerseits eine Bodenbefestigung und andererseits eine Verankerung für zwei Gasmembranen - eine Innen- und eine Außen-Gasmembran 7, 8 - die Dichtungsschicht 4 und die Feuchtigkeitsisolationsschicht 48 des Erdbeckens 19 bereitstellt. Die Innen- und die Außen-Gasmembran 7, 8 sind an ihren äußeren Rändern mittels eines Winkelprofils 71 gemeinsam gasdicht geklemmt. Die äußere Gasmembran 7 dient dem Schutz gegen äußere Einflüsse und wird durch ein zwischen dieser und der inneren Gasmembran 8 eingeleitetes Stützgas von der inneren Gasmembran 8 beabstandet gehalten. Je nach Gas-Füllstand ist die innere Gasmembran 8 angehoben (strichlierte Linie) oder abgesenkt (ausgezogene Linie). Die am Stahlbetonfundament 40 festgelegten Tragegurte 20 bilden ein Tragnetz und sind vorgesehen, um zu verhindern, dass die innere Membran 8 auf den Boden des Erdbeckens 19 absinkt und dabei verschmutzt oder beschädigt wird.

Das Erdbecken 19 wird beispielsweise hergestellt, indem das Stahlbetonfundament 40 im Erdbereich ausgebildete und dann innerhalb des umlaufenden Stahlbetonfundaments 40 das Erdreich so abgetragen wird, dass die Wandschrägen und die Beckensohle des Beckenbodens 3 ausgebildet werden. Danach wird die Schutzfolie 48 gegen Feuchtigkeit und bedarfsweise eine Wärmedämmungsschicht 47 aufgelegt und darauf die gasdichte Dichtungsschicht 4 aufgebracht, die an ihren Rändern durch eine Klemmvorrichtung 72 auf dem Stahlbetonfundament 40 gasdicht geklemmt ist. Um einen besseren Halt der Dichtungsbahn der Dichtungsschicht 40 zu ermöglichten, ist diese über das Stahlbetonfundament 40 hinaus in einen Einbindegraben 55 geführt, in dem die Ränder der Dichtungsschicht 4 nach unten geführt sind. Der Einbindegraben 55 wird danach verfüllt.

Die Dichtungsschicht 4 wird ohne Durchdringungen verlegt. Somit sind keine Durchführungen oder sonstige Durchlässe vorgesehen, welche die Dichtheit der Dichtungsschicht beeinträchtigen könnten.

Damit kann das innerhalb des Erdbeckenfermenters 1 bei der Fermentation entstehende Gas nicht nach außen entweichen.

Die Bedienbrücke 5' überspannt das Erdbecken 19 von der einen zur gegenüberliegenden Seite der Beckenkrone 2 und ist über zumindest zwei Stützen 10 gegenüber dem Beckenboden 3 abgestützt, der in seinem horizontalen Bereich durch einen Betonfundament-Körper 41 gesichert ist. Auf der begehbar ausgeführten Bedienbrücke 5' sind zwei gasdichte Schächte 17 mit Schachtöffnungen ausgebildet, über welche jeweils das senkrechte Führungselement 35, das an seinem unteren Ende auf dem Betonfundament 41 aufgestützt ist, in die Tiefe führt. Die Auflager 152 für die Fixierung des oberen Teils der Führungselemente 35 sind im Bereich der Schachtöffnungen ausgebildet. Auf diesen Führungselementen 35 sind die Rührwerke 9 höhenverstellbär gehalten. Die Schächte 17 ermöglichen die Steuerung und Wartung der Rührwerke 9 bzw. ihre mechanische Ankopplung. Zusätzlich sind nicht dargestellte Durchlässe in der Bedienbrücke 5' und/oder in den Serviceschächten 17 vorgesehen, durch die Zu- und Ableitungen geführt sind.

Weiters ist in der Bedienbrücke 5' ein nicht dargestelltes Sichtfenster ausgenommen, das gasdicht verschließbar ist.

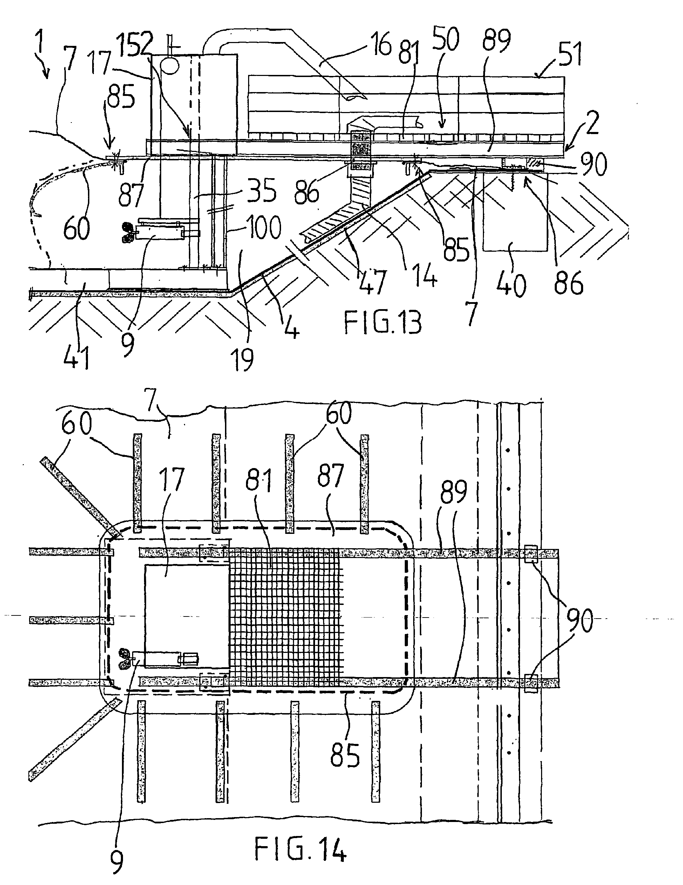

Im Ausführungsbeispiel gemäß Fig.13 und 14 ist ein Bediensteg 50 vorgesehen, der an einem Ende im Bereich der Beckenkrone 2 befestigt ist und mit seinem freien Ende in das Erdbecken 19 ragt. Der Bediensteg 50 ist durch zwei Träger 89 gebildet, die eine Bodenabdeckplatte 87 tragen und die über Stützen 100 gegenüber dem Betonfundament-Körper 41 des Beckenbodens 3 abgestützt sind. Im Bereich der Beckenkrone 2 liegen die Träger 89 auf Lagern 90 auf.

Auf dem Bediensteg 50 ist ein gasdichter Durchlass 86 ausgebildet, durch welchen die Zu- und Ableitung 14 gasdicht in das darunter liegende Erdbecken 19 hindurchgeführt ist. Weiters ist auf dem Bediensteg 50 ein dichtend verschließbarer Service-Schacht 17 mit einer Schachtöffnung angeordnet, durch welche das Führungselement 35 für das Rührwerk 9 hindurch geführt ist, um an der durch den Steg 50 gebildeten Auflagerstelle 152 festgelegt zu sein.

Im oberen Bereich ist der Service-Schacht 17 mit der Gasableitung 16 verbunden, die mit dem Innenraum des Erdbeckenfermenters 1 kommuniziert. Zur Ausführung von Servicearbeiten ist der Bediensteg 50 begehbar und dafür mit einem Laufrost 81 versehen, der an den Seiten durch ein Schutzgeländer 51 gesichert ist.

Der Bediensteg 50 bildet mit seiner Bodenabdeckplatte 87 einen Abschnitt der gasdichten Abdeckung aus, indem die Bodenabdeckplatte 87 eine Öffnung in der Gasmembran 7 verschließt (Fig.14), wobei die Bodenabdeckplatte 87 mit dem Bediensteg 50 gasdicht verbunden ist, sodass der Bediensteg 50 zusammen mit der Gasmembran 7 die gasdichte Abdeckung des Erdbeckens 19 ausbilden. Anstelle einer einteiligen Gasmembran 7 kann diese auch aus mehreren Teilen zusammengesetzt sein.

Die gasdichte Verbindung zwischen der Bodenabdeckplatte 87 und der Gasmembran 7 ist dabei durch einen entlang des Randbereiches der Bodenabdeckplatte 87 des Bedienstegs 50 verlaufende gasdichte Klemmung 85 gebildet, welche die Berandung der Öffnung in der Gasmembran 7 gasdicht einspannt. Um zu verhindern, dass die Gasmembran 7 mit dem Rührwerk 9 in Kontakt kommt und dabei zerstört wird, sind normal zur Bodenabdeckplatte 87 vorstehende Abstandhalter 60, die aus gebogenen Kunststoffstreifen gebildet sind, entlang des Randes der Bodenabdeckplatte 87 angebracht.

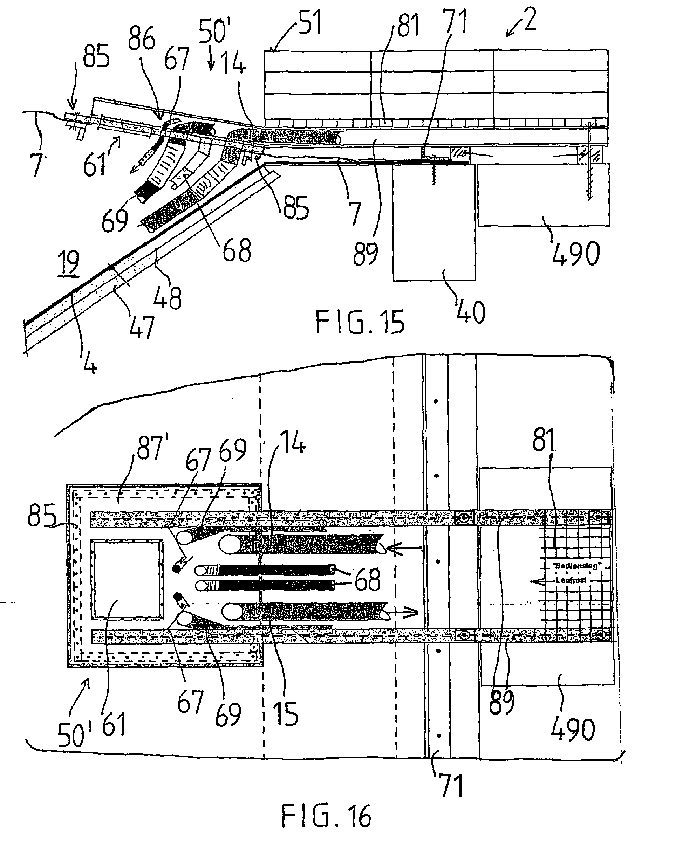

In der Ausführungsform gemäß Fig.15 und 16 ragen die Träger 89 des Bediensteges 50' ohne Stützen in das Erdbecken 19. Für die sichere Verankerung im Bereich der Beckenkrone 2 sorgt ein zusätzliches Widerlager 490, an dem die Träger 89 festgeschraubt sind. Da keine Schächte auf dem Bediensteg 50' ausgebildet sind, genügt eine relativ kleinflächige Bodenabdeckplatte 87', welche anstelle eines Schachtes eine gasdicht verschließbare Bedienöffnung 61 aufweist, über die Sicht bzw. Zutritt in das Erdbecken 19 ermöglicht wird. Im Bereich der Bedienöffnung 61 kann eine Auflagerstelle ausgebildet sein, welche entweder ein Rührwerk lagert oder eine Auflagerstelle für ein Führungselement darstellen kann.

Wie im Ausführungsbeispiel gemäß Fig. 13, 14 verschließt die Bodenabdeckplatte 87' eine Öffnung in der Gasmembran 7 gasdicht und bildet somit zusammen mit der Gasmembran 7 die gasdichte Abdeckung für das Erdbecken 19. Für ausreichende Durchwirbelung des in den gefüllten Erdbeckenfermenter 1 gefüllten Substrats sorgen Gas-Zuleitungen 69 für ein nicht dargestelltes Gasrührwerk. Weiters sind Druckluftzufuhrleitungen 67 über gasdichte Durchlässe durch den Bediensteg 50' hindurch in das Innere des Erdbeckens 19 geführt. Über die Zuleitung 14 wird Substrat, wie z.B. Gülle in den erfindungsgemäßen Erdbeckenfermenter 1 geleitet, oder es kann diese Zuleitung14 für Umpump- oder Spülvorgänge genutzt werden. Über die Ableitung 15 kann das verbrauchte Substrat wieder entnommen werden.

Für die Erhöhung der Fermentationsgeschwindigkeit beispielsweise bei niedrigen Außentemperaturen sind Heizzu- und ableitungen 68 durch den Bediensteg 50' hindurchgeführt, über die dem Erdbecken 19 ein Wärmemedium zuführbar ist.

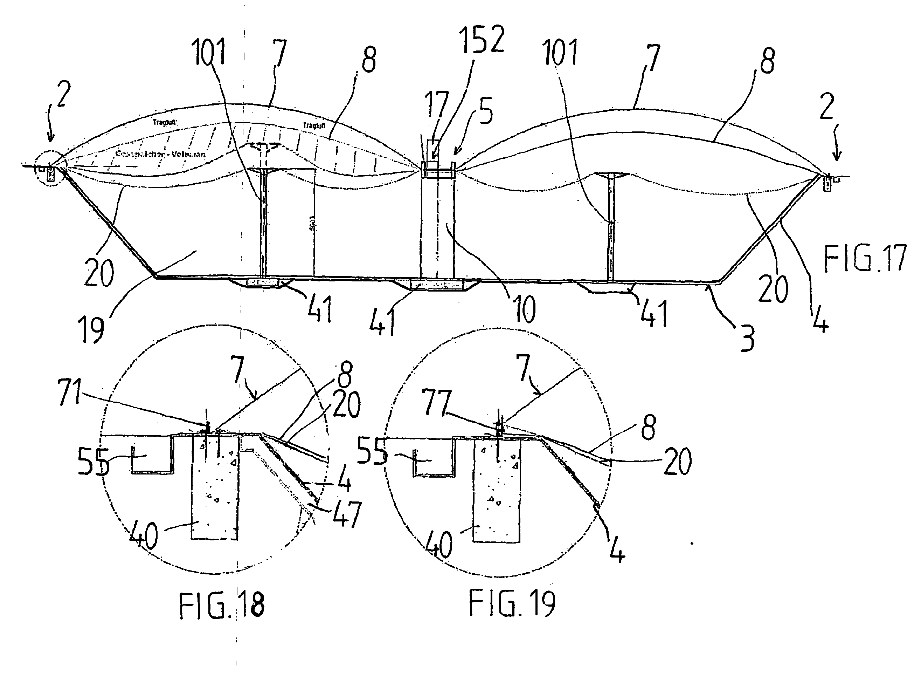

Fig.17 zeigt eine Ausführungsform der Erfindung, bei der wie in Fig.11, 12 und 13 die Bedienbrücke 5 auf halber Länge das Erdbecken 19 überspannt. Zusätzlich zu den beiden Außenmembranteilen 7 sind entsprechende Innenmembranteile 8 ausgebildet, die zusammen mit den Außenmembranteilen entlang der Beckenkrone 2 und entlang der Längsseiten des Bediensteges 5 gasdicht geklemmt sind, wobei zwischen dem linken Außenmembranteil 7 und dem linken Innenmembranteil 8 genauso wie zwischen dem rechten Außenmembranteil 7 und dem rechten Innenmembranteil 8 eine Stützgas-Atmosphäre für die nach außen gewölbte Form der Außenmembran 7 sorgt. Sowohl im linken als auch im rechten Beckenteil sind Stützträger 101 vorgesehen, welche Stützgurte 20 halten und zusammen mit diesen das vollständige Absinken der Innenmembranteile 8 auf beiden Seiten verhindern. Die Stützträger 101 und die Stütze 10 für die Bedienbrücke 5 sind in Fundamenten 41 gesichert. Auf der Bedienbrücke 5 ist ein Schacht 17 mit einer darin befindlichen Schachtöffnung vorgesehen, in deren Bereich eine Auflagerstelle für ein Rührwerk oder für ein Führungselement desselben vorhanden sein kann.

In Fig.18 und 19 sind verschiedene Formen der gasdichten Klemmung der Außen- und Innenmembran 7, 8 auf der Beckenkrone gezeigt, wobei in Fig.18 die Klemmdichtung mittels Winkelprofils 71 und in Fig.19 diese mit einem U-Profil 77 erfolgt.

## Patentansprüche

1. Erdbeckenfermenter (1) mit einem abgesenkten Beckenboden (3), der mit einer Dichtungsschicht (4) abgedeckt ist und ein Erdbecken (19) ausbildet, und an dessen Beckenkrone (2) zumindest eine gasdichte Abdeckung (7, 8) festgelegt ist, wobei Zu- und Ableitungen (14, 15, 16, 67, 68, 69) und zumindest ein Rührwerk (9) vorgesehen sind, wobei zumindest eine Auflagerstelle (151, 152, 153) für das zumindest eine Rührwerk (9) oder für zumindest ein Führungs oder Abstützelement (35) des zumindest einen Rührwerks (9) im Wesentlichen auf der Höhe der Beckenkrone (2) oder darüber und innerhalb des von der Beckenkrone (2) umschlossenen Bereichs vorgesehen ist, **dadurch gekennzeichnet, dass** die zumindest eine Auflagerstelle außerhalb der zumindest einen gasdichten Abdeckung (7, 8) vorgesehen ist, wobei ein Antriebselement des zumindest einen Rührwerks (9) oder das zumindest eine Führungs- oder Abstützelement des zumindest einen Rührwerks (9) gasdicht durch eine Öffnung in der zumindest einen gasdichten Abdeckung (7, 8) hindurchgeführt ist.

2. Erdbeckenfermenter nach Anspruch 1, **dadurch gekennzeichnet, dass** das Führungselement (35) durch eine Führungsschiene gebildet ist, die mit ihrem unteren Ende in einem Fundament (180) am Beckenboden verankert ist und mit ihrem oberen Ende, das durch die gasdichte Abdeckung hindurchgeführt ist, auf der zumindest einen Auflagerstelle (151, 152, 153) abgestützt ist.

3. Erdbeckenfermenter nach Anspruch 1 oder 2, **dadurch gekennzeichnet, dass** die Dichtungsschicht (4) des Beckenbodens (3) durchdringungsfrei ausgeführt ist, und dass die Zu- und Ableitungen (14, 15, 16, 67, 68, 69) oberhalb des Beckenbodens (3) und innerhalb der Beckenkrone (2) in das Innere des Erdbeckens (19) geführt sind.

4. Erdbeckenfermenter nach einem der Ansprüche 1 bis 3, **dadurch gekennzeichnet, dass** eine Bedienbrücke (5, 5') vorgesehen ist, die das Erdbecken (19) überspannt, und dass die zumindest eine Auflagerstelle (153) auf der Bedienbrücke (5, 5') ausgebildet ist.

5. Erdbeckenfermenter nach Anspruch 4, **dadurch gekennzeichnet, dass** in der Bedienbrücke ein oder mehrere Durchlässe vorgesehen sind, durch welche die Zu- und Ableitungen (14, 15,16) und/oder das Führungs- oder Abstützelement und/oder das Antriebselement des Rührwerks (9) gasdicht in das darunter liegende Erdbecken (19) hindurchgeführt sind.

6. Erdbeckenfermenter nach Anspruch 4 oder 5, **dadurch gekennzeichnet, dass** auf der Bedienbrücke (5, 5') zumindest ein dichtend verschließbarer Service-Schacht (17) mit einer Schachtöffnung angeordnet ist, in dem die Auflagerstelle für das zumindest eine Führungs- oder Abstützelement (35) des Rührwerks (9) vorgesehen ist, welches entlang des Führungselements (35) höhenverstellbar ist.

7. Erdbeckenfermenter nach Anspruch 6, **dadurch gekennzeichnet, dass** der Service-Schacht (17) mit einer Zu- oder Ableitung (16) verbunden ist, die mit dem Innenraum des Erdbeckenfermenters (1) kommuniziert

8. Erdbeckenfermenter nach einem der Ansprüche 4 bis 7, **dadurch gekennzeichnet, dass** die Bedienbrücke (5, 5') begehbar ist.

9. Erdbeckenfermenter nach einem der Ansprüche 4 bis 8, **dadurch gekennzeichnet, dass** die Bedienbrücke (5) über zumindest eine Stütze (10) gegenüber dem Beckenboden (3) abgestützt ist

10. Erdbeckenfermenter nach einem der Ansprüche 4 bis 9, **dadurch gekennzeichnet, dass** in der Bedienbrücke ein Sichtfenster ausgenommen ist, das gasdicht verschließbar ist.

11. Erdbeckenfermenter nach einem der Ansprüche 4 bis 10, **dadurch gekennzeichnet, dass** die Bedienbrücke zumindest einen Träger und eine Bodenabdeckplatte umfasst.

12. Erdbeckenfermenter nach einem der Ansprüche 4 bis 11, **dadurch gekennzeichnet, dass** die Bedienbrücke (5, 5') mit ihrer Bodenabdeckplatte einen Abschnitt der gasdichten Abdeckung ausbildet, und dass zumindest eine Gasmembran (7, 8) oder Gasmembranteile (7, 8) mit der Bedienbrücke (5, 5') gasdicht verbunden sind, sodass die Bedienbrücke (5, 5') zusammen mit der Gasmembran (7, 8) oder den Gasmembranteilen die gasdichte Abdeckung des Erdbeckens (19) ausbilden.

13. Erdbeckenfermenter nach Anspruch 12, **dadurch gekennzeichnet, dass** die gasdichte Verbindung zwischen der Bedienbrücke (5, 5') und der zumindest einen Gasmembran (7, 8) oder den Gasmembranteilen (7, 8) durch eine gasdichte Klemmung im Randbereich der Bedienbrücke (5, 5') gebildet ist.

14. Erdbeckenfermenter nach einem der Ansprüche 1 oder 2, **dadurch gekennzeichnet, dass** ein Bediensteg (50, 50') vorgesehen ist, der an einem Ende im Bereich der Beckenkrone (2) befestigt ist und mit seinem freien Ende in das Erdbecken (19) ragt, und dass die zumindest eine Auflagerstelle (151, 152) auf dem Bediensteg (5, 5') ausgebildet ist.

15. Erdbeckenfermenter nach Anspruch 14, **dadurch gekennzeichnet, dass** auf dem Bediensteg (50, 50') ein oder mehrere Durchlässe (86) vorgesehen sind, durch welche die Zu- und Ableitungen (14, 15, 16, 67, 68, 69) gasdicht in das darunter liegende Erdbecken (19) hindurchgeführt sind.

16. Erdbeckenfermenter nach Anspruch 15, **dadurch gekennzeichnet, dass** auf dem Bediensteg (50, 50') zumindest ein dichtend verschließbarer Service-Schacht (17) mit einer Schachtöffnung angeordnet ist, in dem die Auflagerstelle für das zumindest eine Führungs- oder Abstützelement (35) des Rührwerks (9) vorgesehen ist, welches entlang des Führungselements (35) höhenverstellbar ist.

17. Erdbeckenfermenter nach Anspruch 16, **dadurch gekennzeichnet, dass** der Service-Schacht (17) mit einer Zu- oder Ableitung (16) verbunden ist, die mit dem Innenraum des Erdbeckenfermenters (1) kommuniziert.

18. Erdbeckenfermenter nach einem der Ansprüche 14 bis 17, **dadurch gekennzeichnet, dass** der Bediensteg (50, 50') begehbar ist.

19. Erdbeckenfermenter nach einem der Ansprüche 14 bis 18, **dadurch gekennzeichnet, dass** der Bediensteg (50) über zumindest eine Stütze (100) gegenüber dem Beckenboden (3) abgestützt ist.

20. Erdbeckenfermenter nach einem der Ansprüche 14 bis 19, **dadurch gekennzeichnet, dass** im Bediensteg (50') ein Sichtfenster (61) ausgenommen ist, das gasdicht verschließbar ist.

21. Erdbeckenfermenter nach einem der Ansprüche 14 bis 20, **dadurch gekennzeichnet, dass** der Bediensteg (50, 50') zumindest einen Träger (89)und eine Bodenabdeckplatte (87) umfasst.

22. Erdbeckenfermenter nach Anspruch 21, **dadurch gekennzeichnet, dass** der Bediensteg (50, 50') mit seiner Bodenabdeckplatte (87) einen Abschnitt der gasdichten Abdeckung ausbildet, und dass zumindest eine Gasmembran (7, 8) oder Gasmembranteile mit dem Bediensteg (50, 50') gasdicht verbunden sind, sodass der Bediensteg (5, 5') zusammen mit der Gasmembran (7, 8) oder den Gasmembranteilen die gasdichte Abdeckung des Erdbeckens (19) ausbilden.

23. Erdbeckenfermenter nach Anspruch 22, **dadurch gekennzeichnet, dass** die gasdichte Verbindung zwischen dem Bediensteg (50, 50') und der zumindest einen Gasmembran (7, 8) oder den Gasmembranteilen durch eine gasdichte Klemmung (85) im Randbereich der Bodenabdeckplatte (87) gebildet ist.

## Claims

1. A fermenter (1) with an in-ground reservoir having a lowered reservoir floor (3) which is covered by a sealing layer (4) and forms an in-ground reservoir (19), at least one gas-tight cover (7, 8) being fixed to the crest (2) of said reservoir, supplying and draining pipes (14, 15, 16, 67, 68, 69) and at least one agitator (9) being provided, at least one supporting area (151, 152, 153) for the at least one agitator (9) or for at least one guiding or supporting element (35) of the at least one agitator (9) being provided essentially at the level of the reservoir's crest (2) or above said crest and within the area surrounded by the reservoir's crest (2), **characterized in that** said at least one supporting area is provided outside said gas-tight cover (7, 8), a driving element of said at least one agitator (9) or said at least one guiding or supporting element of said at least one agitator (9) being inserted in a gas-tight manner through an opening in said at least one gas-tight cover (7, 8).

2. The fermenter with an in-ground reservoir according to claim 1, **characterized in that** said guiding element (35) is formed by a guiding rail, the lower end of which is fixed to a foundation (180) at the reservoir's floor and the upper end of which is inserted through the gas-tight cover and supported by said at least one supporting area (151, 152, 153).

3. The fermenter with an in-ground reservoir according to claim 1 or claim 2, **characterized in that** said sealing layer (4) of the reservoir's floor (3) is formed without any penetrations and **in that** said supplying and draining pipes (14, 15, 16, 67, 68, 69) are arranged above the reservoir's floor (3) and within the reservoir's crest (2), leading into the interior of the in-ground reservoir (19).

4. The fermenter with an in-ground reservoir according to any one of the claims 1 to 3, **characterized in that** a service bridge (5, 5') is provided, said service bridge crossing the in-ground reservoir (19), and **in that** said at least one supporting area (153) is formed on the service bridge (5, 5').

5. The fermenter with an in-ground reservoir according to claim 4, **characterized in that** one or more passages, through which the supplying and draining pipes (14, 15, 16) and/or the guiding or supporting element and/or the driving element of the agitator (9) are inserted in a gas-tight manner into the in-ground reservoir (19) below, are provided in the service bridge.

6. The fermenter with an in-ground reservoir according to claim 4 or claim 5, **characterized in that** at least one sealingly closable maintenance shaft (17) having a shaft opening is disposed on said service bridge (5, 5'), the supporting area for the at least one guiding or supporting element (35) of the agitator (9) being provided in said shaft, the agitator (9) being vertically adjustable along said guiding element (35).

7. The fermenter with an in-ground reservoir according to claim 6, **characterized in that** said maintenance shaft (17) is connected to a supplying or draining pipe (16) which communicates with the interior of said fermenter (1) with an in-ground reservoir.

8. The fermenter with an in-ground reservoir according to any one of the claims 4 to 7, **characterized in that** said service bridge (5, 5') is weight-bearing.

9. The fermenter with an in-ground reservoir according to any one of the claims 4 to 8, **characterized in that** said service bridge (5) is supported by at least one supporting pillar (10) against the reservoir's floor (3).

10. The fermenter with an in-ground reservoir according to any one of the claims 4 to 9, **characterized in that** a window, which is gas-tightly closable, is provided in the service bridge.

11. The fermenter with an in-ground reservoir according to any one of the claims 4 to 10, **characterized in that** said service bridge comprises at least one beam and a floor covering panel.

12. The fermenter with an in-ground reservoir according to any one of the claims 4 to 11, **characterized in that** said service bridge (5, 5'), together with its floor covering panel, forms a portion of said gas-tight cover, and **in that** at least one gas membrane (7, 8) or gas membrane parts (7, 8) are gas-tightly linked to said service bridge (5, 5'), so that said service bridge (5, 5') forms said gas-tight cover of the in-ground reservoir (19) together with said gas membrane (7, 8) or said gas membrane parts (7, 8).

13. The fermenter with an in-ground reservoir according to claim 12, **characterized in that** the gas-tight connection between the service bridge (5, 5') and the at least one gas membrane (7, 8) or the gas membrane parts (7, 8) is formed by a gas-tight clamping in the edge area of the service bridge (5, 5').

14. The fermenter with an in-ground reservoir according to claim 1 or claim 2, **characterized in that** a service walkway (50, 50') is provided, said walkway being fixed in the area of the reservoir's crest (2) at one end, its free end projecting into the in-ground reservoir (19), and **in that** said at least one supporting area (151, 152) is formed on said service walkway (50, 50').

15. The fermenter with an in-ground reservoir according to claim 14, **characterized in that** one or more passages (86), through which the supplying and draining pipes (14, 15, 16, 67, 68, 69) are inserted in a gas-tight manner into the in-ground reservoir (19) below, is/are provided in said service walkway (50, 50').

16. The fermenter with an in-ground reservoir according to claim 15, **characterized in that** at least one sealingly closable maintenance shaft (17) having a shaft opening is disposed on said service walkway (50, 50'), the supporting area for the at least one guiding or supporting element (35) of the agitator (9) being provided in said shaft, the agitator (9) being vertically adjustable along said guiding element (35).

17. The fermenter with an in-ground reservoir according to claim 16, **characterized in that** said maintenance shaft (17) is connected to a supplying or draining pipe (16) which communicates with the interior of said fermenter (1) with an in-ground reservoir.

18. The fermenter with an in-ground reservoir according to any one of the claims 14 to 17, **characterized in that** said service walkway (50, 50') is weight-bearing.

19. The fermenter with an in-ground reservoir according to any one of the claims 14 to 18, **characterized in that** said service walkway (50) is supported by at least one supporting pillar (100) against the reservoir's floor (3).

20. The fermenter with an in-ground reservoir according to any one of the claims 14 to 19, **characterized in that** a window (61), which is gas-tightly closable, is provided in the service walkway (50').

21. The fermenter with an in-ground reservoir according to any one of the claims 14 to 20, **characterized in that** said service walkway (50, 50') comprises at least one beam (89) and a floor covering panel (87).

22. The fermenter with an in-ground reservoir according to claim 21, **characterized in that** said service walkway (50, 50'), together with its floor covering panel (87), forms a portion of said gas-tight cover, and **in that** at least one gas membrane (7, 8) or gas membrane parts (7, 8) are gas-tightly linked to said service walkway (50, 50'), so that said service walkway (50, 50') forms said gas-tight cover of the in-ground reservoir (19) together with said gas membrane (7, 8) or said gas membrane parts (7, 8).

23. The fermenter with an in-ground reservoir according to claim 22, **characterized in that** the gas-tight connection between the service walkway (50, 50') and the at least one gas membrane (7, 8) or the gas membrane parts (7, 8) is formed by a gas-tight clamping (85) in the edge area of the floor covering panel (87).

## Revendications

1. Fermenteur (1) à réservoir en terre, le fond (3) du réservoir étant abaissé et couvert d'une couche d'étanchéité (4), formant un réservoir en terre (19), au moins une couverture (7, 8), qui est étanche au gaz, étant fixée à la couronne (2) dudit réservoir, des conduites d'alimentation et d'évacuation (14, 15, 16, 67, 68, 69) et au moins un agitateur (9) étant prévus, au moins un endroit d'appui (151, 152, 153) pour ledit au moins un agitateur (9) ou pour au moins un élément de guidage ou de support (35) dudit au moins un agitateur (9) étant prévu sensiblement à l'hauteur de la couronne (2) du réservoir ou là-dessus et à l'intérieur de l'endroit entouré par ladite couronne (2) du réservoir, **caractérisé en ce que** ledit au moins un endroit d'appui est prévu en dehors dudit au moins une couverture (7, 8) étanche au gaz, un élément de commande dudit au moins un agitateur (9) ou ledit au moins un élément de guidage ou d'appui dudit au moins un agitateur (9) étant passé à travers une ouverture dudit au moins une couverture (7, 8) étanche au gaz.

2. Fermenteur à réservoir en terre selon la revendication 1, **caractérisé en ce que** ledit élément de guidage (35) est formé par une glissière de guidage, le bout inférieur de laquelle étant ancré dans une fondation (180) au fond du réservoir et le bout supérieur de laquelle passant à travers la couverture étanche au gaz et étant supporté par ledit au moins un endroit d'appui (151, 512, 153).

3. Fermenteur à réservoir en terre selon la revendication 1 ou 2, **caractérisé en ce que** ladite couche d'étanchéité (4) du fond (3) du réservoir est réalisée sans pénétrations et **en ce que** lesdites conduites d'alimentation et d'évacuation (14, 15, 16, 67, 68, 69) sont introduites dans l'intérieur du réservoir en terre (19) au dessus du fond (3) du réservoir et à l'intérieur de la couronne (2) du réservoir.

4. Fermenteur à réservoir en terre selon l'une des revendications 1 à 3, **caractérisé en ce qu'**un pont de service (5, 5'), qui enjambe le réservoir en terre (19), est prévu et **en ce que** ledit au moins un endroit d'appui (153) est formé sur ledit pont de service (5, 5').

5. Fermenteur à réservoir en terre selon la revendication 4, **caractérisé en ce qu'**un ou plusieurs passages, à travers duquel/desquels les conduites d'alimentation et d'évacuation (14, 15, 16) et/ou l'élément de guidage ou de support et/ou l'élément de commande de l'agitateur (9) sont introduits d'une manière étanche au gaz dans le réservoir en terre (19) sous-jacent, est/sont prévu/s dans le pont de service (5, 5').

6. Fermenteur à réservoir en terre selon la revendication 4 et 5, **caractérisé en ce qu'**au moins une cheminée de service (17), qui peut être fermée d'une manière étanche et qui a une ouverture, est disposée au pont de service (5, 5'), l'endroit d'appui pour ledit au moins un élément de guidage ou d'appui (35) de l'agitateur (9), qui est réglable en hauteur le long de l'élément de guidage (35), étant prévu dans ladite cheminée de service (17).

7. Fermenteur à réservoir en terre selon la revendication 6, **caractérisé en ce que** ladite cheminée de service (17) est reliée à une conduite d'alimentation ou d'évacuation (16) qui est en communication avec l'intérieur du fermenteur (1) à réservoir en terre.

8. Fermenteur à réservoir en terre selon l'une des revendications 4 à 7, **caractérisé en ce que** le pont de service (5, 5') est praticable.

9. Fermenteur à réservoir en terre selon l'une des revendications 4 à 8, **caractérisé en ce que** le pont de service (5) est supporté par au moins un support (10) par rapport au fond (3) du réservoir.

10. Fermenteur à réservoir en terre selon l'une des revendications 4 à 9, **caractérisé en ce qu'**une fenêtre d'inspection, qui peut être fermée d'une manière étanche au gaz, est prévue dans le pont de service.

11. Fermenteur à réservoir en terre selon l'une des revendications 4 à 10, **caractérisé en ce que** le pont de service comprend au moins une poutre et une plaque de couverture.

12. Fermenteur à réservoir en terre selon l'une des revendications 4 à 11, **caractérisé en ce que** le pont de service (5, 5') et sa plaque de couverture forment une partie de la couverture étanche au gaz et **en ce qu'**au moins une membrane à gaz (7, 8) ou des parties de la membrane à gaz (7, 8) est/sont reliée/s au pont de service (5, 5') d'une manière étanche au gaz, ainsi que le pont de service (5, 5') et la membrane à gaz (7, 8) ou les parties de la membrane à gaz forment la couverture étanche au gaz couvrant le réservoir en terre (19).

13. Fermenteur à réservoir en terre selon la revendication 12, **caractérisé en ce que** la jonction étanche au gaz entre le pont de service (5, 5') et ladite au moins une membrane à gaz (7, 8) ou les parties de la membrane à gaz (7, 8) est formée par un serrage étanche au gaz dans la partie de bord du pont de service (5, 5').

14. Fermenteur à réservoir en terre selon la revendication 1 ou 2, **caractérisé en ce qu'**une passerelle de service (50, 50') est prévue, un bout de laquelle étant fixé à l'endroit de la couronne (2) du réservoir, le bout libre surplombant le réservoir en terre (19), et **en ce qu'**au moins un endroit d'appui (151, 152) est formé sur la passerelle de service (50, 50').

15. Fermenteur à réservoir en terre selon la revendication 14, **caractérisé en ce que** qu'un ou plusieurs passages (86), à travers duquel/desquels les conduites d'alimentation et d'évacuation (14, 15, 16, 67, 68, 69) sont introduites d'une manière étanche au gaz dans le réservoir en terre (19) sous-jacent, est/sont prévu/s dans la passerelle de service (50, 50').

16. Fermenteur à réservoir en terre selon la revendication 15, **caractérisé en ce qu'**au moins une cheminée de service (17), qui peut être fermée d'une manière étanche et qui a une ouverture, est disposée à la passerelle de service (50, 50'), l'endroit d'appui pour ledit au moins un élément de guidage ou d'appui (35) de l'agitateur (9), qui est réglable en hauteur le long de l'élément de guidage (35), étant prévu dans ladite cheminée de service (17).

17. Fermenteur à réservoir en terre selon la revendication 16, **caractérisé en ce que** ladite cheminée de service (17) est reliée à une conduite d'alimentation ou d'évacuation (16) qui est en communication avec l'intérieur du fermenteur (1) à réservoir en terre.

18. Fermenteur à réservoir en terre selon l'une des revendications 14 à 17, **caractérisé en ce que** la passerelle de service (50, 50') est praticable.

19. Fermenteur à réservoir en terre selon l'une des revendications 14 à 18, **caractérisé en ce que** la passerelle de service (5) est supportée par au moins un support (100) par rapport au fond (3) du réservoir.

20. Fermenteur à réservoir en terre selon l'une des revendications 14 à 19, **caractérisé en ce qu'**une fenêtre d'inspection (61), qui peut être fermée d'une manière étanche au gaz, est prévue dans la passerelle de service.

21. Fermenteur à réservoir en terre selon l'une des revendications 14 à 20, **caractérisé en ce que** la passerelle de service (50, 50') comprend au moins une poutre (89) et une plaque de couverture (87).

22. Fermenteur à réservoir en terre selon la revendication 21, **caractérisé en ce que** la passerelle de service (50, 50') et sa plaque de couverture (87) forment une partie de la couverture étanche au gaz et **en ce qu'**au moins une membrane à gaz (7, 8) ou des parties de la membrane à gaz est/sont reliée/s à la passerelle de service (50, 50') d'une manière étanche au gaz, ainsi que la passerelle de service (50, 50') et la membrane à gaz (7, 8) ou les parties de la membrane à gaz forment la couverture étanche au gaz couvrant le réservoir en terre (19).

23. Fermenteur à réservoir en terre selon la revendication 22, **caractérisé en ce que** la jonction étanche au gaz entre la passerelle de service (50, 50') et ladite au moins une membrane à gaz (7, 8) ou les parties de la membrane à gaz (7, 8) est formée par un serrage (85) étanche au gaz dans la partie de bord de la plaque de couverture (87).
